# EUROPEAN PATENT SPECIFICATION

(11) **EP 2 027 844 B1**
(45) Date of publication and mention of the grant of the patent: **27.11.2013**
(21) Application number: 08252753.2
(22) Date of filing: 20.08.2008
(51) Int. Cl.: A61G 7/018

(54) **Proximity activiation of voice operation of hospital bed**
Proximitätsaktivierung eines Sprachvorgangs eines Krankenhausbettes
Activation de proximité de la commande vocale d'un lit d'hôpital

(30) Priority: 20.08.2007 US 956902 P; 18.08.2008 US 193560
(43) Date of publication of application: 25.02.2009
(73) Proprietor: Hill-Rom Services, Inc., Batesville, IN 47006 (US)
(72) Inventor: Dixon, Steven Alan, Cincinnati, OH 45247 (US)
(74) Representative: Findlay, Alice Rosemary

(56) References cited:
- EP-A- 1 149 571
- WO-A-01/85085
- WO-A-01/86575
- WO-A-03/018087
- WO-A-2008/042121
- US-A- 5 335 313
- US-A1- 2008 097 177
- US-A1- 2008 189 783

## Description

The present disclosure is related to patient-support apparatuses. More specifically, the present disclosure is related to patient-support apparatuses with sensors configured to sense when a caregiver is proximate to the patient-support apparatus and to configure the patient-support apparatus for the caregiver to provide voice operation of the patient-support apparatus by the caregiver.

Patient-support apparatuses such as hospital beds and procedural tables are becoming much more complex. The functionality of the equipment includes therapeutic functions as well as patient-comfort and positioning functions. As patients become more acute, various caregivers are delivering care at the patient-support apparatus. In some embodiments, the patient-support apparatus is capable of delivering therapies such as pulmonary therapies via a mattress, for example.

Patient-support apparatus movement functions are known to be capable of being locked-out via user interface controls. However, there is no provision for preventing the controls from being locked-out completely based on the identity of the caregiver. Because it is necessary to keep the operation of a patient-support apparatus simple and efficient, traditional locking techniques such as passwords and key systems are not widely accepted.

In addition, each caregiver may have a different level of authorization for changing operational parameters of various functions of a patient-support apparatus. A lab technician may be qualified to reposition a patient and take patient weight, but not authorized to change mattress settings. On the other hand a primary care nurse is likely to be authorized to change any operating parameters.
WO 01/85085 discloses a remote control system for hospital beds in which bed functions can be controlled by remote controls carried by caregivers. The bed can detect proximity of a remote control or a caregiver and subsequently activate particular bed controls.
WO 01/86575 discloses a patient monitoring system that collates data from several devices into a single record. The system includes a receiver for detecting RFID signals from badges, and a voice recognition module configured to provide inputs to a control system.

The present invention is defined in the appended independent claims to which reference should now be made. Preferred embodiments may include the following features, alone or in any combination.

A patient-support apparatus comprises a control system, a receiver configured to detect a signal from a badge when the badge is proximate the patient-support apparatus, and a voice recognition according to claim 1.

The system may be configured to activate the voice recognition module when the receiver detects the signal from the badge. In some embodiments, the control system activates a patient data acquisition function.

The voice recognition module may include a voice-to-text function configured to convert voice information to data that is input to the control system. In some embodiments, the patient-support apparatus further may include a display. The control system may be configured to alter the display to show data being acquired.

In some embodiments, the patient-support apparatus may include a lift system and the voice recognition module may be configured to provide an input to the control system to operate the lift system. At least one inactive function is activated when the receiver detects a signal from the badge. The function activated is associated with a unique identifier in the signal from the badge. The function activated may be a scale function, a mattress function, a motorized function, or a patient data acquisition function.

In some embodiments, the control system may include a user input and the control system may be configured to check for a signal from the badge upon activation of the user input. For example, the user input may be a foot pedal, a discrete switch, or a portion of a touchscreen display.

The voice recognition module may be configured to activate a patient data acquisition function, and the voice recognition module may include a voice-to-text function which is operable to process voice information from a user to convert the voice information to data. The control system may be operable to log data. In addition, the control system may be configured to transfer logged patient data to a hospital information system external to the patient-support apparatus.

A method of operating a multi-function patient-support apparatus including a control system, a voice recognition module, and a wireless receiver comprises monitoring the wireless receiver to determine if a wireless signal has been received. The method also includes determining the status of a user of the patient-support apparatus based on information in the signal. The method still further includes changing the state of one of the functions of the multi-function patient-support apparatus from an inactive state to an active state.

The method comprises determining the function to be changed from an inactive state to an active state by determining that the user is authorized to operate the function based on the status of the user. The method still further comprises determining the status of the user from a unique identifier included in the wireless signal. The method may still yet further comprise communicating with a hospital information system to determine the status of the user. When communicating with a hospital information system, the method may comprise comparing the unique identifier to a database of users to determine the status of the user.

Monitoring the wireless receiver may be commenced upon activation of a user input on the patient-support apparatus. The function activated may be one of a motorized function, a scale function, a mattress function, or a patient data logging function.

When the function activated is a patient data logging function, the method may further comprise transferring logged patient data to a hospital information system.

In some embodiments, the method may further comprise monitoring voice information to determine an input to the control system. Still yet, the method may include converting voice information to text data to be logged in the patient logging function.

The invention will now be further described by way of example with reference to the accompanying drawings, in which:

Fig. 1 is a perspective view of a patient-support apparatus and a caregiver wearing a proximity badge according to the present disclosure;

Fig. 2 is a block diagram of patient-support apparatus of Fig. 1;

Fig. 3 is a diagrammatic view of an user interface panel of the patient-support apparatus of Fig. 1, the user interface panel in a first condition;

Fig. 4 a diagrammatic view of the user interface panel of Fig. 3, the user interface panel in a second condition; and

Fig. 5 is a diagrammatic view of the user interface panel of Fig. 3, the user interface panel in a third condition.

A patient-support apparatus includes a proximity identification system which determines when a caregiver is proximate the patient-support apparatus and modifies the functionality of the patient-support apparatus.

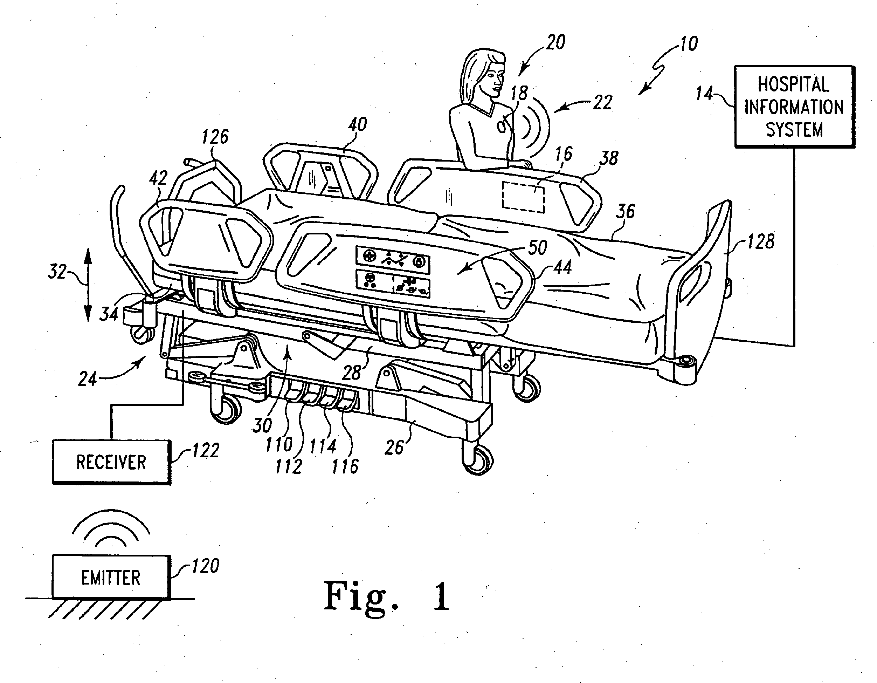

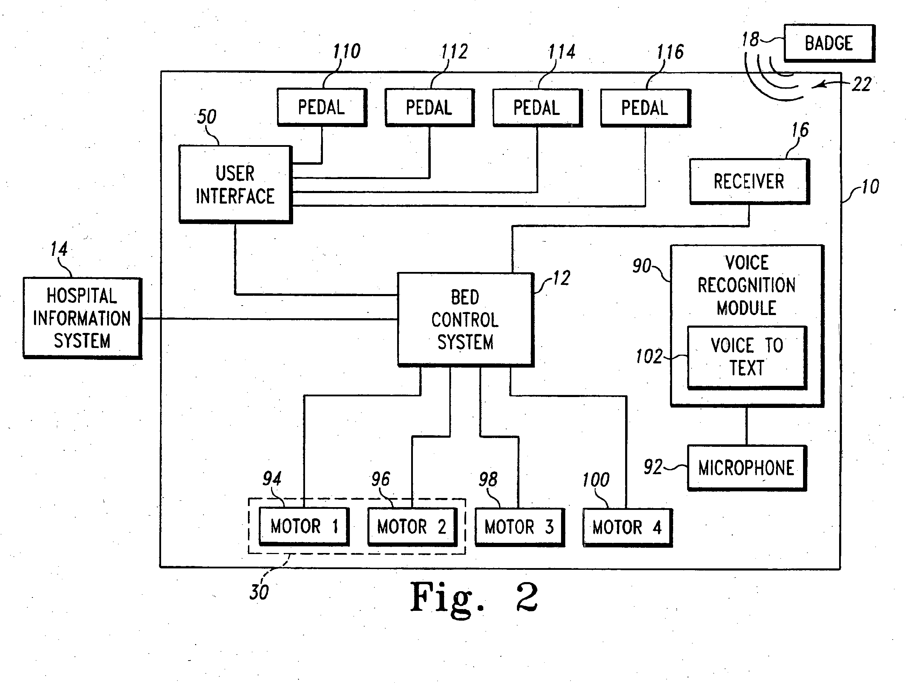

Referring to Fig. 1, a patient support apparatus is illustratively embodied as a hospital bed 10 and includes a bed control system 12 (shown diagrammatically in Fig. 2) which includes circuitry to control operation of the bed 10 and communication between the bed 10 and a hospital information system 14 the bed 10. Bed 10 includes an receiver 16 coupled to the bed 10 and configured to receive a signal from a proximity badge 18 worn by a caregiver 20 to detect that the caregiver 20 is within range of the bed 10 and to alter the functionality of the bed 10 when the caregiver 20 is present. In the illustrative embodiment, the badge 18 transmits a wireless communication signal 22 which is received by the receiver 16.

Bed 10 includes a frame structure 24 having a lower frame 26 and an upper frame 28 which is movable relative the lower frame 26. A lift system 30 is operable to raise and lower the upper frame 28 relative to the lower frame 26 as indicated by arrow 32. A deck 34 is supported on upper frame 28 and is movable relative to the upper frame 28 to change the position of a patient positioned on the bed 10. A mattress 36 is supported on deck 34. Mattress 36 includes a number of air bladders and foam components and various control parameters of the mattress 36 allow the mattress 36 to provide various therapies to a patient supported on the mattress 36. For example, mattress 36 is operable to provide rotation and percussion therapy to the patient. Mattress 36 is also configured to allow a caregiver to deflate a portion of the mattress 36 to assist in moving a patient such as for turning the patient to change bed linens or to stiffen a portion of the mattress 36 to assist a patient in exiting the bed 10.

Deck 34 is articulated such that various portions of the deck 34 may be moved to raise, for example, the head, thighs, or feet of a patient. Movement of the various deck portions and lift system 30 is controlled by the bed control system 12.

Bed 10 includes a number of siderails 38, 40, 42, 44 which are illustratively supported on upper frame 28 and movable from the raised positions shown in Fig. 1 to lowered positions.

Proximity badge 18 is configured to have limited range communication such that the badge has to be within a minimum distance before the receiver 16 recognizes the signal from the proximity badge 18. In the illustrative embodiment of Fig. 1, proximity badge 18 includes a radio frequency transmitter communicates with receiver 16 via communication signal 22 which is, illustratively, a radio frequency signal. In other embodiments, proximity badge 18 may comprise a transmitter in the form of an infrared emitter and receiver 16 may be configured as an infrared receiver such that the communication signal 22 is transferred from the emitter to the receiver as an infrared signal through a line of sight. In the illustrative embodiment, the minimum distance for communication between badge 18 and receiver 16 is about 6 feet. In other embodiments, the range of communication may be greater or less than 6 feet.

Bed 10 is connected to the hospital information system 14 such that information available on the hospital information system 14 may be shared with the bed 10. For example, the hospital information system 14 may be configured such that the capabilities and authorizations of various caregivers is maintained on the hospital information system 14. Each caregiver wears a proximity badge 18 having a unique identifier associated with the caregiver so that the unique identifier is transmitted via the communication signal 22 to the receiver 16. The bed control system 12 is configured to communicate with the hospital information system 14 to determine the authorization of the caregiver and thereby configure the functionality of the bed 10 to correspond to the caregiver. For example, each caregiver is preauthorized to perform certain activities associated with the operation of the bed 10. While some caregivers may be authorized to operate all functions of the bed 10, other caregivers may have limited authorization. A primary nurse, for example, may be permitted to operate all functions of the bed 10. A lab technician may only be permitted to activate motor controls to reposition the patient for blood draws. Still yet, a respiratory therapist may be authorized to change certain operating parameters of the mattress 36.

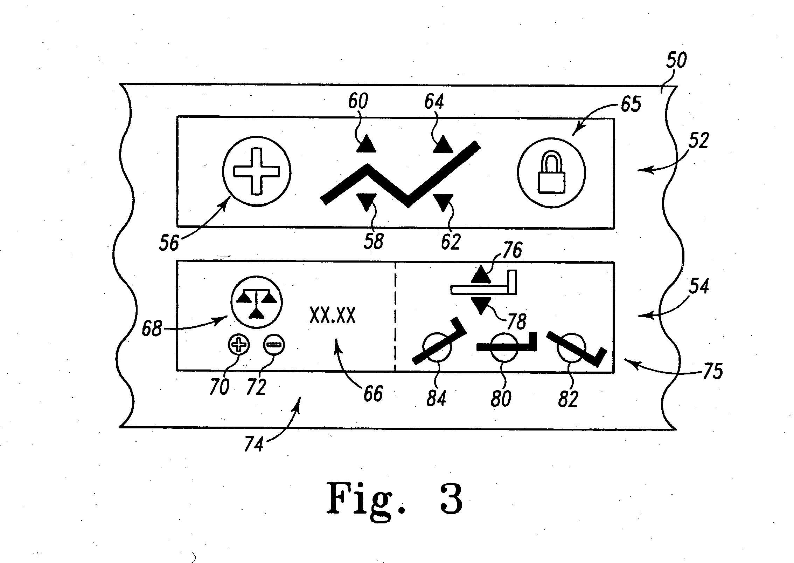

In an illustrative embodiment shown in Fig. 3, a user interface 50 mounted on siderail 44 includes a first panel 52 and a second panel 54. The first panel 52 includes basic operations that include a nurse call switch 56, knee articulation inputs 58 and 60, and head articulation inputs 62 and 64. The first panel 52 also includes a lock-out input 65. Second panel 54 includes a scale operation section 74 including a weight display 66, a scale activation input 68, and adjustment inputs 70 and 72. Second panel 54 also includes a hi-lo operation section 75 including bed raise and lower inputs 76 and 78, bed flat input 80, and bed tilt and reverse tilt inputs 82 and 84. In the illustrative embodiment of Fig. 3, inputs 56, 58, 60, 62, 64 and 65 are all discrete membrane switches which are always visible to a user. Second panel 54 is illustratively a touchscreen display which is operable display various graphical images and sense the touch of a user who contacts portions of the display.

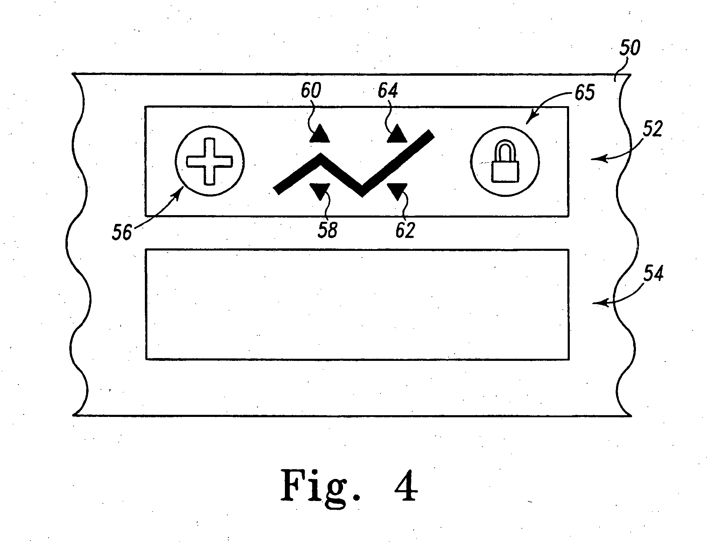

Referring now to Fig. 4, second panel 54 is blank with no icons or information displayed. This may occur, for example, when no caregiver having authority to use the scale system of bed 10 or lift system 30 of the bed is detected to be within the minimum distance from the bed 10. Thus, the scale operation section 74 and hi-lo operation section 75 are inactive and not available for a user.

Bed control system 12 includes software which modifies the operation of the second panel 54 to permit an appropriately trained caregiver access to the user input scale operation section 74 and hi-lo operation section 75. By automatically recognizing the presence of a caregiver and reconfiguring the second panel 54, the caregiver is able to operate the patient-support apparatus 10 to adjust certain functions. Similarly, a person who is not a trained or authorized to operate certain features the patient-support apparatus 10 cannot access protected controls.

In a similar manner, the lack of a proximity badge 18 of an authorized user may serve to lock-out or prevent activation of various discrete user inputs such as inputs 56, 58, 60, 62, 64 and 65. Thus, while the inputs 56, 58, 60, 62, 64 and 65 are constantly available, the underlying functionality may not activate unless an appropriate user is proximate to the bed 10.

Proximity badge 18 is also operable to enable voice activated controls of patient-support apparatus 10. Illustratively, patient-support apparatus 10 includes a voice recognition module 90. Voice recognition module 90 is connected to a microphone 92 and is configured to monitor signals from the microphone 92 when active. Voice recognition module processes signals from the microphone 92 into commands transmitted to the bed control system 12 of the patient-support apparatus 10. As shown in Fig. 2, patient-support apparatus 10 includes four motors 94, 96, 98 and 100. Bed control system 12 controls operation of the motors 94, 96, 98 and 100 to move various portions of bed 10. Voice recognition module 90 is configured to receive a voice command from a caregiver and to process the command to provide an input to bed control system 12.

For example, a caregiver, such as a nurse, for example, may walk within an acceptable proximity of the patient-support apparatus 10 and utter "start head up." The voice recognition module is operable to recognize the "start head up" command and initiate a signal to the bed control system 12 that is the equivalent of the head up input 62 to raise a portion of the deck 34 so that the head a patient is raised. Other similar commands may be utilized as well.

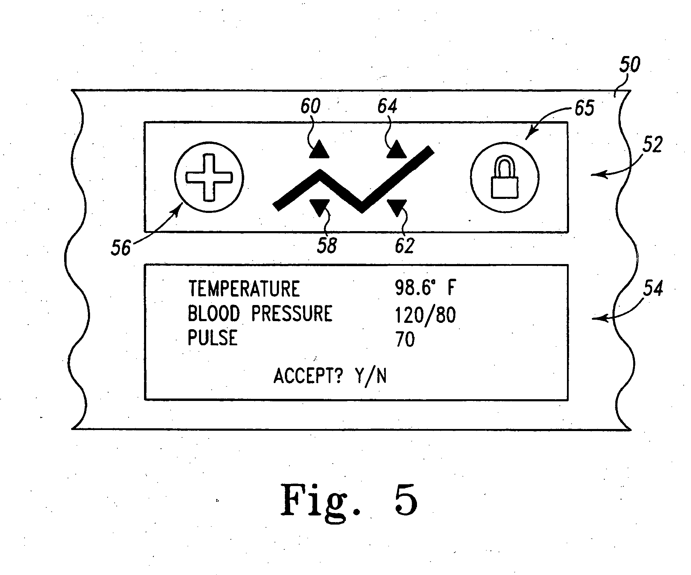

Additionally, the voice recognition module 90 is configured to allow hands-free logging of patient data. For example, a caregiver may activate the voice recognition module 90 by proximity and state "vital signs." The voice recognition module 90 will then activate a data logging function through the bed control system 12. Once the bed control system 12 data logging function is active, a display, such as second panel 54 may be activated to display data to be logged as shown in Fig. 5. For example, the term "vital signs" may cause the bed control system 12 to recognize that the caregiver is taking the patient's vital signs. The caregiver may then recite vital signs which are displayed on the second panel 54 for the caregiver's approval. For example, the caregiver may state "temperature 98.6, blood pressure 120 over 80, pulse 70" and the corresponding data would is displayed on the second panel 54. Display of the data on the on the second panel 54 is facilitated by a voice-to-text function 102 of the voice recognition module 90 which converts the voice data transmitted by the microphone 92 to text data input to the bed control system 12. The bed control system 12 acquires the text data and utilizes software to display the appropriate text on the panel 54 which is representative of the data acquired by the control system 12.

Once the caregiver is finished inputting vital signs, the caregiver may review the data displayed on the second panel 54 for accuracy. If the data is accurate, the caregiver may accept the data by uttering "accept data," which causes the vital signs data to be accepted by the bed control system 12. The data may be logged within the bed control system 12 and the data may be transferred to the hospital information system 14 by the bed control system 12. The connection between the bed 10 and the hospital information system 14 provides a unique identifier from the bed 10 which transmits the bed identification to the hospital information system 14. This allows the data to be linked to a particular patient who is associated with the bed 10 within the hospital information system 14.

Bed 10 includes a number of foot pedals 110, 112, 114, and 116 which are configured to operate various functions of the bed 10. Rather than utilizing a proximity badge 18, the voice recognition module 90 may become active if a foot pedal 110, 112 114, or 116 is activated or a particular input is activated. A pedal similar to pedals 110, 112, 114, or 116 or an input similar to inputs 56, 58, 60, 62, 64 and 65 may be dedicated to activating the voice recognition module 90. A caregiver may then operate functions of bed 10 or log data as described above. The voice recognition module 90 may become dormant after some period if the voice recognition module 90 has not received voice input from the microphone.

In the illustrative embodiment, data the bed control system 12 communicates with the hospital information system 14. In some embodiments, the bed 10 may include a network and each of the electrical components may be coupled to the network to transfer data between the modules and to control communication between the modules. Any of a number of communication protocols may be employed to control the communication. For example, in some embodiments a controller area network (CAN) may be utilized including a serial bus for the physical layer and the CANOpen protocol for communications.

While in the illustrative embodiment the receiver 16 is located on the patient-support apparatus 10, the receiver 16 may be positioned proximate to, but not directly on the patient-support apparatus. For example, in some embodiments receiver 16 may be mounted to the head wall proximate to the patient-support apparatus 10. In other embodiments, the antenna may be mounted to an IV pole, a headboard 126 of the patient-support apparatus 10, a footboard 128 of the patient-support apparatus 10, mattress 36 supported on the patient-support apparatus 10, or on some structure of a room in which the patient-support apparatus 10 is located. For example, the receiver 16 could be positioned in the ceiling or on the floor proximate to the patient-support apparatus 10. In embodiments where the receiver 16 is spaced apart from the patient-support apparatus 10, receiver 16 may be in wireless communication with the patient-support apparatus 10 utilizing an infrared emitter 120 to communicate with an infrared receiver 122 mounted on the patient-support apparatus 10 as shown in Fig. 1. In particular, this configuration may be employed when the patient-support apparatus 10 is docked to a particular location in a room thereby facilitating a line of sight between the infrared emitter and the infrared receiver because of the known location of the components on the patient-support apparatus 10.

It should be understood that any of a number of inputs such as inputs 56, 58, 60, 62, 64 and 65 may be active based on the presence of a particular caregiver. Also, bed 10 may include multiple displays similar to panel 54 and various inputs may be displayed or activated based on the detection of a particular caregiver. For example, a display may be menu driven so that multiple functions are available through a particular display and the caregiver may scroll through the various available functions. In addition, data logging features may be activated based on the particular caregiver who is proximate to the bed 10 or detected by the receiver 22.

## Claims

1. A patient-support apparatus (10) comprising
a control system (12),
a receiver (16) configured to detect a signal (22) from a badge (18) when the badge (18) is proximate the patient-support apparatus (10), and
a voice recognition module (90) configured to provide Inputs to the control system (12) to control at least one function of the patient-support apparatus (10), wherein the control system (12) is configured to activate at least one inactive function of the patient support apparatus (10) when the receiver (16) detects a signal (22) from the badge (18), a unique identifier in the signal (22) from the badge (18) being indicative of the status of a user wearing the badge, **characterized in that** the control system (12) is operative to determine the function to be changed from an inactive state to an active state by determining that the user is authorized to operate the function based on the status of the user.

2. The patient-support apparatus (10) of claim 1, wherein the control system (12) is configured to activate the voice recognition module (90) when the receiver (16) detects the signal (22) from the badge (18).

3. The patient-support apparatus (10) of claim 2, wherein the control system (12) activates a patient data acquisition function.

4. The patient-support apparatus (10) of claim 3, wherein the voice recognition module (90) includes a voice-to-text function (102) configured to convert voice information to data that is input to the control system (12).

5. The patient-support apparatus (10) of either claim 3 or claim 4, wherein the patient-support apparatus (10) further includes a display (54) and wherein the control system (12) is configured to alter the display (54) to show data being acquired.

6. The patient-support apparatus (10) of any preceding claim, wherein the patient-support apparatus (10) includes a lift system (30) and the voice recognition module (90) is configured to provide an input to the control system (12) to operate the lift system (30).

7. The patient-support apparatus (10) of any preceding claim, wherein the control system (12) includes a user input and the control system (12) is configured to check for a signal (22) from the badge (18) upon activation of the user input.

8. The patient-support apparatus (10) of claim 7 wherein the user input is a foot pedal (110, 112, 114, 116) or a discrete switch (56, 58, 60, 62, 64, 65), or a portion of a touchscreen display (54).

9. The patient-support apparatus (10) of any preceding claim, wherein the voice recognition module (90) is configured to activate a patient data acquisition function, and wherein the voice recognition module (90) includes a voice-to-text function (102) which is operable to process voice information from a user to convert the voice information to data and the control system (12) is operable to log the data.

10. The patient-support apparatus (10) of claim 9, wherein the control system (12) is configured to transfer logged patient data to a hospital information system (14) external to the patient-support apparatus (10).

11. A method of operating a multi-fonction patient-support apparatus (10) including a control system (12), a voice recognition module (90), and a wireless receiver (16), the method comprising:
monitoring the wireless receiver (16) to determine if a wireless signal (22) has been received,
upon receipt of a wireless signal (22), determining the status of a user of the patient-support apparatus (10) based on information in the signal (22),
changing the state of one of the functions of the multi-function patient-support apparatus (10) from an inactive state to an active state, **characterized by** determining the function to be changed from an inactive state to an active state by determining that the user is authorized to operate the function based on the status of the user.

12. The method of claim 11, wherein the monitoring of the wireless receiver (16) is commenced upon activation of a user input on the patient-support apparatus (10).

13. The method of either claim 11 or claim 12, wherein the function activated is a patient data logging function.

14. The method of claim 13, further comprising transferring logged patient data to a hospital information system (14).

15. The method of any one of claims 11 to 14, further comprising monitoring voice information to determine an input to the control system (12).

16. The method of claim 15, further comprising converting voice information to text data to be logged in the patient logging function.

## Patentansprüche

1. Patientenunterstützungsvorrichtung (10) mit einem Steuerungssystem (12), mit einem Empfänger (16) zur Erfassung eines Signals (22) von einem Ausweis (18), wenn sich der Ausweis (18) in der Nähe der Patientenunterstützungsvorrichtung (10) befindet, und mit einem Spracherkennungsmodul (90), das zur Generierung von Eingaben für das Steuerungssystem (12) konfiguriert ist, um mindestens eine Funktion der Patientenunterstützungsvorrichtung (10) zu steuern, wobei die Konfiguration des Steuerungssystems (12) dazu dient, mindestens eine inaktive Funktion der Patientenunterstützungsvorrichtung (10) zu aktivieren, wenn der Empfänger (16) ein Signal (22) von einem Ausweis (18) erfasst, wobei ein unverwechselbares Erkennungszeichen in dem vom Ausweis (18) stammenden Signal (22) als Anhaltspunkt für den Status eines die Ausweis tragenden Benutzers gilt, **dadurch gekennzeichnet, dass** das Steuerungssystem (12) dahingehend wirksam wird, dass die von einem inaktiven Zustand in einen aktiven Zustand zu ändernde Funktion dadurch bestimmt wird, dass festgestellt wird, dass der Benutzer ausgehend vom Status des Benutzers befugt ist, die Funktion zu aktivieren.

2. Patientenunterstützungsvorrichtung (10) nach Anspruch 1, wobei das Steuerungssystem (12) so konfiguriert ist, dass das Spracherkennungsmodul (90) aktiviert wird, wenn der Empfänger (16) das vom Ausweis (18) stammende Signal (22) erfasst.

3. Patientenunterstützungsvorrichtung (10) nach Anspruch 2, wobei das Steuerungssystem (12) eine Funktion zur Erfassung von Patientendaten aktiviert.

4. Patientenunterstützungsvorrichtung (10) nach Anspruch 3, wobei das Spracherkennungsmodul (90) eine Sprach-zu-Text-Funktion (102) umfasst, die zur Umwandlung von Sprachinformationen in Daten konfiguriert ist, welche in das Steuerungssystem (12) eingegeben werden.

5. Patientenunterstützungsvorrichtung (10) nach entweder Anspruch 3 oder Anspruch 4, wobei die Patientenunterstützungsvorrichtung (10) des Weiteren ein Display (54) umfasst und wobei das Steuerungssystem (12) konfiguriert ist, um das Display (54) so zu verändern, dass die erfassten Daten angezeigt werden.

6. Patientenunterstützungsvorrichtung (10) nach irgendeinem der vorhergehenden Ansprüche, wobei die Patientenunterstützungsvorrichtung (10) ein Hebesystem (30) umfasst und wobei das Spracherkennungsmodul (90) so konfiguriert ist, dass zum Wirksamwerden des Hebesystems (30) eine Eingabe zum Steuerungssystem (12) erfolgt.

7. Patientenunterstützungsvorrichtung (10) nach irgendeinem der vorhergehenden Ansprüche, wobei das Steuerungssystem (12) eine Benutzereingabe umfasst und wobei das Steuerungssystem (12) so konfiguriert ist, dass bei Aktivierung der Benutzereingabe eine Prüfung auf ein vom Ausweis (18) stammendes Signal (22) vorgenommen wird.

8. Patientenunterstützungsvorrichtung (10) nach Anspruch 7, wobei die Benutzereingabe ein Fußpedal (110, 112, 114, 116) oder ein Einzelschalter (56, 58, 60, 62, 64, 65), oder ein Teil eines Touchscreen-Displays (54) ist.

9. Patientenunterstützungsvorrichtung (10) nach irgendeinem der vorhergehenden Ansprüche, wobei das Spracherkennungsmodul (90) zur Aktivierung einer Funktion zur Erfassung von Patientendaten konfiguriert ist und wobei das Spracherkennungsmodul (90) eine Sprach-zu-Text-Funktion (102) umfasst, die benutzt werden kann, um Sprachinformationen von einem Benutzer so zu verarbeiten, dass die Sprachinformationen in Daten umgewandelt werden, und wobei das Steuerungssystem (12) zur Protokollierung der Daten wirksam werden kann.

10. Patientenunterstützungsvorrichtung (10) nach Anspruch 9, wobei das Steuerungssystem (12) konfiguriert ist, um protokollierte Patientendaten zu einem außerhalb der Patientenunterstützungsvorrichtung (10) befindlichen Krankenhaus-Informationssystem (14) zu übertragen.

11. Verfahren zum Betrieb einer Multifunktions-Patientenunterstützungsvorrichtung (10) einschließlich eines Steuerungssystems (12), eines Spracherkennungsmoduls (90) und eines Funkempfängers (16), wobei das Verfahren umfasst:
- Überwachung des Funkempfängers (16) zwecks Feststellung, ob ein Funksignal (22) empfangen worden ist,
- nach Empfang eines Funksignals (22) Feststellung des Status eines Benutzers der Patientenunterstützungsvorrichtung (10) auf der Grundlage von im Signal (22) enthaltenen Informationen,
- Änderung des Status einer der Funktionen der Multifunktions-Patientenunterstützungsvorrichtung (10) von einem inaktiven Zustand in einen aktiven Zustand, **gekennzeichnet durch** die Bestimmung der von einem inaktiven Status in einen aktiven Status zu ändernden Funktion **durch** Feststellung, dass der Benutzer ausgehend vom Status des Benutzers zur Benutzung der Funktion befugt ist.

12. Verfahren nach Anspruch 11, wobei mit der Überwachung des Funkempfängers (16) nach Aktivierung einer Benutzereingabe an der Patientenunterstützungsvorrichtung (10) begonnen wird.

13. Verfahren nach entweder Anspruch 11 oder Anspruch 12, wobei es sich bei der aktivierten Funktion um eine Funktion zur Protokollierung von Patientendaten handelt.

14. Verfahren nach Anspruch 13, mit des Weiteren der Übertragung von protokollierten Patientendaten zu einem Krankenhausinformationssystem (14).

15. Verfahren nach irgendeinem der Ansprüche 11 bis 14, mit des Weiteren der Überwachung von Sprachinformationen zur Feststellung einer Eingabe an das Steuerungssystem (12).

16. Verfahren nach Anspruch 15, mit des Weiteren der Umwandlung von Sprachinformationen in Textdaten, die unter der Patientenprotokollierungsfunktion protokolliert werden sollen.

## Revendications

1. Appareil de support de patient (10) comprenant
un système de commande (12),
un récepteur (16) configuré pour détecter un signal (22) en provenance d'un badge (18) lorsque le badge (18) est à proximité de l'appareil de support de patient (10), et
un module de reconnaissance vocale (90) configuré pour fournir des entrées au système de commande (12) pour commander au moins une fonction de l'appareil de support de patient (10), dans lequel le système de commande (12) est configuré pour activer au moins une fonction inactive de l'appareil de support de patient (10) lorsque le récepteur (16) détecte un signal (22) en provenance du badge (18), un identificateur unique dans le signal (22) en provenance du badge (18) indiquant le statut d'un utilisateur portant le badge, **caractérisé en ce que** le système de commande (12) est fonctionnel pour déterminer la fonction à changer d'un état inactif à un état actif en déterminant que l'utilisateur est autorisé à exploiter la fonction sur la base du statut de l'utilisateur.

2. Appareil de support de patient (10) selon la revendication 1, dans lequel le système de commande (12) est configuré pour activer le module de reconnaissance vocale (90) lorsque le récepteur (16) détecte le signal (22) en provenance du badge (18).

3. Appareil de support de patient (10) selon la revendication 2, dans lequel le système de commande (12) active une fonction d'acquisition de données de patient.

4. Appareil de support de patient (10) selon la revendication 3, dans lequel le module de reconnaissance vocale (90) comprend une fonction voix-texte (102) configurée pour convertir des informations vocales en données qui sont entrées dans le système de commande (12).

5. Appareil de support de patient (10) selon la revendication 3 ou la revendication 4, dans lequel l'appareil de support de patient (10) comprend en outre un afficheur (54) et dans lequel le système de commande (12) est configuré pour modifier l'afficheur (54) pour montrer des données qui sont acquises.

6. Appareil de support de patient (10) selon l'une quelconque des revendications précédentes, dans lequel l'appareil de support de patient (10) comprend un système de levage (30) et le module de reconnaissance vocale (90) est configuré pour fournir une entrée au système de commande (12) pour faire fonctionner le système de levage (30).

7. Appareil de support de patient (10) selon l'une quelconque des revendications précédentes, dans lequel le système de commande (12) comprend une entrée d'utilisateur et le système de commande (12) est configuré pour vérifier un signal (22) en provenance du badge (18) lors de l'activation de l'entrée d'utilisateur.

8. Appareil de support de patient (10) selon la revendication 7, dans lequel l'entrée d'utilisateur est une pédale (110, 112, 114, 116) ou un interrupteur discret (56, 58, 60, 62, 64, 65), ou une partie d'un afficheur à écran tactile (54).

9. Appareil de support de patient (10) selon l'une quelconque des revendications précédentes, dans lequel le module de reconnaissance vocale (90) est configuré pour activer une fonction d'acquisition de données de patient, et dans lequel le module de reconnaissance vocale (90) comprend une fonction voix-texte (102) qui est exploitable pour traiter des informations vocales en provenance d'un utilisateur pour convertir les informations vocales en données et le système de commande (12) est exploitable pour consigner les données.

10. Appareil de support de patient (10) selon la revendication 9, dans lequel le système de commande (12) est configuré pour transférer des données de patient consignées à un système d'information hospitalier (14) externe à l'appareil de support de patient (10).

11. Procédé d'exploitation d'un appareil de support de patient multifonction (10) comprenant un système de commande (12), un module de reconnaissance vocale (90), et un récepteur sans fil (16), le procédé comprenant :
la surveillance du récepteur sans fil (16) pour déterminer si un signal sans fil (22) a été reçu,
lors de la réception d'un signal sans fil (22), la détermination du statut d'un utilisateur de l'appareil de support de patient (10) sur la base d'informations dans le signal (22),
le changement de l'état d'une des fonctions de l'appareil de support de patient multifonction (10) d'un état inactif à un état actif, **caractérisé par** la détermination de la fonction à changer d'un état inactif à un état actif en déterminant que l'utilisateur est autorisé à exploiter la fonction sur la base du statut de l'utilisateur.

12. Procédé selon la revendication 11, dans lequel la surveillance du récepteur sans fil (16) est commencée lors de l'activation d'une entrée d'utilisateur sur l'appareil de support de patient (10).

13. Procédé selon la revendication 11 ou la revendication 12, dans lequel la fonction activée est une fonction de consignation de données de patient.

14. Procédé selon la revendication 13, comprenant en outre le transfert de données de patient consignées à un système d'information hospitalier (14).

15. Procédé selon l'une quelconque des revendications 11 à 14, comprenant en outre la surveillance d'informations vocales pour déterminer une entrée dans le système de commande (12).

16. Procédé selon la revendication 15, comprenant en outre la conversion d'informations vocales en données texte à consigner dans la fonction de consignation de patient.
